Europäisches Patentamt

European Patent Office

Office européen des brevets

(19)

(11) **EP 1 144 586 B1**

(12) **EUROPÄISCHE PATENTSCHRIFT**

(45) Veröffentlichungstag und Bekanntmachung des Hinweises auf die Patenterteilung:
**28.08.2002 Patentblatt 2002/35**

(21) Anmeldenummer: **00903502.3**

(22) Anmeldetag: **05.01.2000**

(51) Int Cl.⁷: **C12M 1/34**

(86) Internationale Anmeldenummer:
**PCT/DE00/00071**

(87) Internationale Veröffentlichungsnummer:
**WO 00/044876 (03.08.2000 Gazette 2000/31)**

(54) **VERFAHREN, GEFÄSS UND VORRICHTUNG ZUR ÜBERWACHUNG DER STOFFWECHSELAKTIVITÄT VON ZELLKULTUREN IN FLÜSSIGEN MEDIEN**

METHOD, VESSEL AND DEVICE FOR MONITORING METABOLIC ACTIVITY OF CELL CULTURES IN LIQUID MEDIA

PROCEDE, RECIPIENT ET DISPOSITIF POUR LA SURVEILLANCE DE L'ACTIVITE METABOLIQUE DE CULTURES CELLULAIRES DANS DES MILIEUX LIQUIDES

(84) Benannte Vertragsstaaten:
**AT BE CH CY DE DK ES FI FR GB GR IE IT LI LU MC NL PT SE**

(30) Priorität: **29.01.1999 DE 19903506**

(43) Veröffentlichungstag der Anmeldung:
**17.10.2001 Patentblatt 2001/42**

(73) Patentinhaber: **Institut für Chemo- und Biosensorik Münster e.v.**
**48149 Münster (DE)**

(72) Erfinder:
• **KATERKAMP, Andreas**
  **D-48149 Münster (DE)**
• **KEY, Göran**
  **D-49084 Osnabrück (DE)**
• **CHEMNITIUS, Gabriele**
  **D-48153 Münster (DE)**

(74) Vertreter: **Pfenning, Meinig & Partner GbR**
**Gostritzer Strasse 61-63**
**01217 Dresden (DE)**

(56) Entgegenhaltungen:
**EP-A- 0 810 281    WO-A-87/00023**
**WO-A-88/06287    WO-A-95/03254**
**WO-A-99/47922    US-A- 4 548 907**
**US-A- 5 601 997**

EP 1 144 586 B1

**Beschreibung**

Die Erfindung betrifft ein Verfahren zur Überwachung der Stoffwechselaktivität von Zellen in flüssigen Medien, ein hierfür besonders geeignetes Gefäß und entsprechende Vorrichtung zur Durchführung des Verfahrens.

In den Gefäßen sind zu kultivierende Zellen sowie ein flüssiges Medium enthalten, wobei es sich bei letzterem, um eine herkömmlich gegebenenfalls der verwendeten Zellen entsprechende Nährlösung handelt. Die erfindungsgemäße Lösung kann für die verschiedensten Zellen und die verschiedensten Untersuchungen, insbesondere im pharmakologischen Bereich eingesetzt werden, wobei die Stoffwechselaktivität der Zellen über einen längeren Zeitraum beobachtet werden kann. Es kann z.B. eine Wirkungsüberwachung bei Cytotoxizitäts- und Biokompatibilitätstests durchgeführt werden, oder die Optimierung der Kulturbedingungen für die Produktion von Biomolekülen.

Die Hauptnährstoffquelle für Zellkulturen ist üblicherweise Glukose, die durch aerobe Glukolyse zu Laktat oder oxidativ unter Sauerstoffverbrauch und Kohlendioxid-Bildung umgewandelt werden kann. Dabei wirken sich viele Einflüsse der Physiologie einer Zelle in ihrer Stoffwechselaktivität aus, so daß auch der Sauerstoffverbrauch entsprechend schwanken kann. Ausgehend vom Zusammenhang Stoffwechselaktivität der Zellen in bezug zum Sauerstoffverbrauch und z.B. dem Glukose, L-Glutamin Verbrauch oder der Erzeugung von Laktat kann aus deren Kenntnis auf den Zustand der überwachten Zellen und demzufolge auf den Einfluß der jeweiligen Kulturbedingungen geschlossen werden.

Auf diesen Erkenntnissen aufbauend ist von Lisa Randers-Eichhorn u.a. in "Noninvasive Oxygen Measurements and Mass Transfer Cosiderations in Tissue Culture Flasks", veröffentlicht in Biotechnology and Bioengineering, Vol. 51, Seiten 466 bis 478 beschrieben worden, wie der Sauerstoffverbrauch von in T-Flaschen kultivierten Zellen durch optische Messung bestimmt werden kann. Dort wird vorgeschlagen, Fluoreszenzindikatoren enthaltende Sensormembranen unmittelbar auf dem Flaschenboden und im Gasraum oberhalb der Nährlösung in einer solchen T-Flasche anzuordnen. Bei der Sauerstoffkonzentrationsmessung mit solchen Sensormembranen wird das bekannte physikalische Phänomen der Fluoreszenzlöschung bekannter Fluoreszenzfarbstoffe, wie z.B. Ruthenium (II)-Komplexe, infolge von Sauerstoffeinfluß ausgenutzt, wobei sich die jeweilige Fluoreszenzintensität, bei konstanter Anregung entsprechend der Sauerstoffkonzentration bzw. des Sauerstoffpartialdruckes verändert. Zur Bestimmung der Sauerstoffkonzentration kann die jeweilige Fluoreszenzintensität unmittelbar aber auch die Fluoreszenzlebensdauer gemessen werden und anhand einer bekannten Kalibration die Sauerstoffkonzentration bestimmt werden.

Die in diesem Dokument beschriebene Meßanordnung, insbesondere die Anordnung der Sensormembran auf dem Boden der T-Flaschen und die vernachlässigte Bestimmung einiger wichtiger Einflußgrößen, eignet sich nicht dazu, über den Sauerstoffverbrauch auf die Stoffwechselaktivität der kultivierten Zellen zu schließen.

Der Eintrag von Sauerstoff in die Nährlösung erfolgt zum größten Teil über die Grenzfläche der Nährlösung zum Gasraum, hier also zum Gasraum in der T-Flasche und der Verbrauch erfolgt durch die auf dem Boden der T-Flasche befindlichen Zellen. Die maximal mögliche Sauerstoffkonzentration, die in der Nährlösung erreicht werden kann, ist die Sauerstoffsättigungskonzentration $C_{sat}$ (in mg/l), die nach der Gleichung

$$C_{sat} = ([p - \gamma * p_w(T)]/p_o * \alpha(T) * X_{o2}$$

vom Gesamtgasdruck p (in mbar), der relativen Feuchte $\gamma$ (in Werten von 0 bis 1, wobei 1 einem Wert von 100 % und 0 einem von 0% entspricht), dem Wasserdampfpartialdruck $p_w(T)$ (in mbar) in Abhängigkeit von der Temperatur T, dem Molenbruch des Sauerstoffs $X_{02}$ im Gasraum der T-Flasche,e dem Bunsen'schen Absorptionskoeffizienten $\alpha(T)$ (in mg/l) in Abhängigkeit von der Temperatur T und dem Normaldruck $p_o = 1013$ mbar abhängt. Dabei ist vorausgesetzt, daß der Gasraum und die Nährlösung die gleiche Temperatur besitzen und der Gasraum mit atmosphärischer Luft, deren chemische Zusammensetzung hinlänglich bekannt ist, gefüllt wurde. Diese Voraussetzungen sind üblicherweise in Brutschränken, in denen Zellen kultiviert werden, gegeben. Diese Sauerstoffsättigungskonzentration stellt sich direkt unterhalb der Oberfläche in der Nährlösung ein. Von dort wird der Sauerstoff durch unterschiedliche Effekte, wie Diffusion und/oder Konvektion zu den am Boden befindlichen Zellen transportiert. Zwei Größen sind in einem solchen System maßgebend. Zum einen ist dieses die Verbrauchsrate $k_v$, mit der der Sauerstoff durch die Zellen verbraucht wird und zum anderen die Transportrate $k_T$, mit der der Sauerstoff zu den Zellen transportiert wird. Beide Größen zusammen sind verantwortlich dafür, daß von der Oberfläche der Nährlösung zum Boden mit Zellen ein Sauerstoffgradient entsteht. Ist nun die Verbrauchsrate geringfügig kleiner oder gleich groß wie die Transportrate, so wird mit der Sauerstoffmembran am Boden unterhalb der Zellen eine Sauerstoffkonzentration vom Wert Null bestimmt. Die Zellen leiden in diesem Fall aber nicht an einer Sauerstoffversorgung, da noch genügend Sauerstoff zu den Zellen transportiert wird, dieser aber nicht mehr zur Sensormembran unterhalb der Zellen gelangt und entsprechend auch nicht mehr gemessen werden kann. Wenn die Verbrauchsrate weiter ansteigt, z.B. durch Vermehren der Zellen, und die Verbrauchsrate größer wird als die Transportrate, so kann dieses mit der Sauerstoffmembran am Boden unterhalb der Zellen auch nicht mehr beobachtet werden. Weiterhin ist die Sauerstoffsättigungskonzentration neben der Verbrauchs- und Transportrate in Abhängigkeit vom Gesamtgasdruck, der Feuchte, der Temperatur und dem Molenbruch des Sauerstoff bzw. dem Sauerstoffpartialdruck im Gasraum der T-Flasche eine sehr wichtige Größe. Eine Änderung verursacht eine Änderung des Sauerstoffgradienten und damit eine Änderung der Sauerstoffkonzentration an einer beliebigen Stelle zwischen den Zellen und der Oberfläche der Nährlösung. Da die Parameter Gesamtdruck, Feuchte und Temperatur in der genannten Dokumentation nicht bestimmt oder kontrolliert wurden, ist nicht auszuschließen, daß durch Schwankungen in diesen Parametern die Meßergebnisse verfälscht wurden.

Die WO-A-99/47922 beschreibt ein Verfahren zur Uberwachung der biologischen Aktivität von den flüssigen Medien kultivierten Zellen bei dem die Zellen im Inneren von Kanälen einer Mikromatrix, die sich in einer mit Lichtwellenleiter ausgestatteten Perfusionskammer befindet, kultiviert. Die Änderungen der Sauerstoffkonzentration im Perfusionsmedium wird dabei anhand von Fluoreszenzmessungen vorgenommen.

Die US 4,548,907 offenbart einen fluoreszenzoptischen Sensor zur Bestimmung eines Analyten (z. B. $CO_2$ in einer Lösung).

Die US 5,601,979 offenbart ein Verfahren zur Untersuchung der biologischen Aktivität in flüssigen Medien, wobei ein Gefäß verwendet wird, das in zwei Bereiche unterteilt ist, wobei in dem einen Teil fluoreszenzmarkierte Zellen vorliegen. Die Zellen können in den benachbarten Bereich durch eine Membran diffundieren, wo sie optisch detektiert werden.

Es ist daher Aufgabe der Erfindung, Möglichkeiten vorzugeben, mit der die Überwachung des Sauerstoffverbrauches und damit die Stoffwechselaktivität kultivierter Zellen kostengünstig und mit erhöter Genauigkeit erreicht werden kann.

Erfindungsgemäß wird diese Aufgabe mit den Merkmalen des Anspruchs 1 für ein Verfahren und den Merkmalen des Anspruchs 11 für ein geeignetes Gefäß gelöst. Vorteilhafte Ausgestaltungsformen und Weiterbildungen der Erfindung, ergeben sich mit den in den untergeordneten Ansprüchen genannten Merkmalen.

Die erfindungsgemäße Lösung geht nunmehr davon aus, daß Zellen in Gefäßen unter Verwendung eines flüssigen Mediums, wobei es sich hier in der Regel um eine entsprechende Nährlösung handelt, kultiviert werden und deren Stoffwechselaktivität über die Messung der Sauerstoffkonzentration, an einem Ort im flüssigen Medium zwischen den Sauerstoff verbrauchenden Zellen und dem für den Sauerstoffeintrag in das flüssige Medium maßgebenden Teil, hier die Oberfläche der Nährlösung, erfolgt. Dabei wird die Sauerstoffsättigungskonzentration im flüssigen Medium anhand von Vergleichsmessungen in einem Zellkulturgefäß ohne Zellen und/oder über die Bestimmung der Parameter Druck, Feuchte, Temperatur und bei bekannter und konstanter chemischer Zusammensetzung des umgebenden Gasraumes, hier die Molenbrüche der Gaskomponenten bzw. deren Partialdrücke in atmosphärischer Luft bestimmt. Aus dem Vergleich zwischen der Sauerstoffsättigungskonzentration als Sollwert und der Sauerstoffkonzentration an einer Stelle des Sauerstoffgradienten im Gefäß mit den kultivierten Zellen als Istwert wird der auf den Sauerstoffverbrauch und damit auf die Stoffwechselaktivität der Zellen geschlossen.

Für die Überwachung können hierbei, im Gegensatz zum Stand der Technik, ohne weiteres Gefäße verwendet werden, die über eine Öffnung verfügen, so daß die Oberfläche des flüssigen Mediums von der umgebenden Atmosphäre beeinflußt werden kann. Eine solche Öffnung kann in bestimmten Fällen aber auch mit einer zumindest für Sauerstoff permeablen Membran abgedeckt bzw. abgeschlossen sein, so daß beispielsweise ein Eindringen unerwünschter Keime verhindert werden kann.

Die Sauerstoffkonzentration wird, bevorzugt optisch mit einer hierfür geeigneten Sensormembran gemessen, deren optische Eigenschaften sich in Abhängigkeit von der jeweiligen Sauerstoffkonzentration verändern. Es sollte sich also in einem entsprechenden Gefäß, zumindest eine geeignete Sensormembran befinden, die so angeordnet ist, daß sie oberhalb der auf dem Gefäßboden kultivierten Zellkulturen, jedoch unterhalb der Oberfläche des flüssigen Mediums angeordnet ist.

Verbrauchen die auf dem Gefäßboden kultivierten Zellen Sauerstoff, wird sich entsprechend die Sauerstoffkonzentration im flüssigen Medium verringern und die tatsächlich mit der Sensormembran gemessene Sauerstoffkonzentration wird durch den Sauerstoffverbrauch infolge Stoffwechselaktivität und der Sauerstoffmenge, die infolge des aufgetretenen Sauerstoffkonzentrationsgradienten wieder in das flüssige Medium hinein gelangt, bestimmt.

Da die Gesetzmäßigkeiten für die Sättigungskonzentration von Sauerstoff in flüssige Medien relativ genau bekannt sind, besteht die Möglichkeit, bei Berücksichtigung bekannter Parameter, hier insbesondere der entsprechenden Temperatur, Druck und Feuchte die entsprechende Sauerstoffsättigungskonzentration in einem flüssigen Medium zu berechnen, so daß dieser berechnete Sauerstoffkonzentrationswert einem Wertevergleich mit der tatsächlich gemessenen Sauerstoffkonzentration für die Bewertung des Sauerstoffkonsums bzw. der Stoffwechselaktivität der Zellkulturen unterzogen werden kann.

Es besteht aber auch die Möglichkeit, eine Rerferenzmessung durchzuführen, wobei ein zweites Gefäß verwendet wird, in dem lediglich ein Nährmedium, das bezüglich der Sauerstoffeindiffusionseigenschaften mit den verwendeten Nährlösungen völlig identisch ist, verwendet wird. In einem solchen Referenzgefäß ist wiederum, bevorzugt an gleicher Stelle eine entsprechende Sensormembran angeordnet, mit der die unbeeinflußte Sauerstoffkonzentration gemessen werden kann. Auch die so gemessene Referenzsauerstoffkonzentration kann mit der durch Stoffwechselaktivität beeinflußten Sauerstoffkonzentration einem entsprechenden Wertevergleich unterzogen werden, um die Stoffwechselaktivität der kultivierten Zellen zu beurteilen.

Selbstverständlich kann ein solcher Wertevergleich auch gemeinsam für die berechnete Sauerstoffkonzentration und das Sauerstoffkonzentrationsmeßsignal im Referenzgefäß mit der unter von kultivierten Zellen moderierten Stoffwechsel gemessenen Sauerstoffkonzentration gleichzeitig durchgeführt werden.

Die Aussagekraft bei der Bewertung der Stoffwechselaktivität von kultivierten Zellen kann außerdem erhöht werden, wenn zusätzlich Sensormembranen in den Gefäßen und diese hier wiederum so angeordnet werden, wie die sauerstoffsensitiven Membrane, die eine Oxidoreductase auf einer sauerstoffsensitiven Membran enthalten und mit denen die Sauerstoffkonzentrationsänderung durch Substratumsatz des Enzyms gemessen werden kann.

Günstigerweise sollten diese beiden verschiedenen Sensormembranen im zumindest nahezu gleichen Abstand von den Zellkulturen innerhalb des flüssigen Mediums angeordnet sein. Zur Ermittlung der Konzentration des Oxidoreductasesubstrats und damit der Stoffwechselaktivität werden die Floreszenzsignale der Sauerstoffmembran und der zweiten Sauerstoffmembran, die mit der zusätzlichen Membran belegt ist, verglichen. Dabei kann es notwendig sein, das Signal der ersten Sensormembran von dem Signal der zweiten Sensormembran abzuziehen, um so die Sensorantwort auf den enzymatischen Versuch des Sauerstoffs zu erhalten und damit die Substratkonzentration zu bestimmen. Dabei kann auch die Einführung eines Faktors vorteilhaft sein, durch den die unterschiedlichen Sauerstofftransportverhältnisse an den beiden Sensormembranen berücksichtigt sind. Es kann außerdem auch notwendig sein, zur Auswertung des Signals des enzymatischen Sensors je nach Sauerstoffkonzentration in der Lösung verschiedene Kalibrationskurven zu verwenden, da Sauerstoff ein Cosubstrat der enzymatischen Reaktion ist. In jedem Fall kann durch eine Bestimmung der Enzymaktivität die Stoffwechselaktivität der Zellen im weiteren unabhängig vom Sauerstoffverbrauch der kultivierten Zellen bestimmt werden.

Da sich die Stoffwechselaktivität der kultivierten Zellen relativ langsam über größere Zeiträume verändert, genügt es, die jeweiligen Konzentrationen in größeren Zeitabständen, beispielsweise in Intervallen von mehreren Minuten zu messen, um mit ausreichender Genauigkeit, die Stoffwechselaktivität der jeweiligen Zellkulturen zu überwachen, was zur Folge hat, daß sich der erforderliche Aufwand für einen geeigneten Meßaufbau durch einen möglichen Mulitplexbetrieb verringern läßt.

Da die Konzentration vorteilhaft optisch gemessen werden soll, ist es erforderlich, Gefäße zu verwenden, die zumindest in bestimmten Bereichen optisch transparent sind, so daß die entsprechende Lichtintensitätsänderung, beispielsweise mit Lichtwellenleitern (Glasfasern) und einem geeigneten optischen Sensor gemessen werden kann. Dabei muß ein solcher Lichtwellenleiter nicht unmittelbarer Bestandteil eines verwendeten Gefäßes oder mit diesem unmittelbar verbunden sein, sondern er kann so angeordnet und ausgerichtet sein, daß er lediglich mit seiner Apertur den Bereich oder Teile einer Sensormembran erfassen kann. Meßort und Meßgefäß können demzufolge ohne weiteres örtlich voneinander getrennt sein.

Es besteht weiterhin die Möglichkeit, wie bereits mit der Möglichkeit der multiplexen Messung angedeutet, mehrere Gefäße, in denen gleiche oder verschiedene Zellen kultiviert werden, gemeinsam zu überwachen, wobei diese jeweils einzeln nacheinander durch entsprechende Schaltung zumindest eines Multiplexers berücksichtigt werden können. Es kann daher eine quasi ortsaufgelöste Messung von Konzentrationen durchgeführt werden. Es können z.B. Sensormembranen verwendet werden, die sauerstoffkonzentrationsabhängig ihre Absorptions- bzw. Reflexionseigenschaften ändern. Es besteht aber auch die Möglichkeit, aufbauend auf bekannten Lösungen, das Phänomen der Fluoreszenzlöschung auszunutzen und Sensormembranen zu verwenden, in denen bekannte Fluoreszenzfarbstoffe enthalten sind, die in der Lage sind, bei Anregung mit Licht bestimmter Wellenlängen zu fluoreszieren, wobei die Wellenlänge des Anregungslichtes und die Wellenlänge des Fluoreszenzlichtes unterschiedlich sind.

Im letztgenannten Fall kann die Konzentration einmal durch direkte Messung der jeweiligen Fluoreszenzintensität gemessen werden. Günstiger ist es aber, die Fluoreszenzlebensdauer zu bestimmen, da in diesem Fall die Alterung und demzufolge auch das bekannte Ausbleichverhalten die Meßgenauigkeit nicht beeinflußt.

Die sauerstoffsensitiven Sensormembranen können mittels verschiedenster Techniken auch nachträglich in, für die erfindungsgemäße Lösung verwendbare Gefäße eingebracht werden. Solche Sensormembranen können durch Dispensieren, Sprühen, Tauchen oder auch Kleben lokal gezielt aufgebracht werden. Geeignete Anbringungsorte in solchen Gefäßen sind z.B. die Gefäßinnenwand in einem bestimmten Abstand vom Gefäßboden und besonders geeignet sind über die Gefäßbodenfläche hinausragende podestartige Elemente, auf deren oberer Stirnfläche eine entsprechende Sensormembran ausgebildet bzw. aufgebracht werden kann. In diesem Fall sind zumindest die podestartigen Elemente aus einem für die relevanten Wellenlängen transparenten Material gebildet, so daß das entsprechende Monitoring von unten durch den Gefäßboden erfolgen kann. Die übrigen Gefäßbestandteile müssen dann nicht zwingend aus einem transparenten Material bestehen.

In einem erfindungsgemäß verwendbaren Gefäß können in einem Abstand voneinander zwei solcher podestartigen Elemente angeordnet sein, auf denen einmal eine ausschließlich sauerstoffsensitive Sensormembran aufgebracht ist und zum anderen eine solche, die mit einer enzymatischen Oxidase beschichteten Membran gegenüber dem flüssigen Medium abgeschlossene Zusatzmembran ausgebildet ist, aufgebracht sein kann.

Besonders günstig ist es, wenn beispielsweise eine größere Anzahl von Proben gleichzeitig überwacht werden soll, ein erfindungsgemäßes Gefäß, analog zu den bekannten Mikrowellplatten auszubilden, bei dem in einer solchen Mikrowellplatte eine größere Anzahl von Aufnahmeräumen (Kavitäten) für die zu kultivierenden Zellen mit dem flüssigen Medium in mehreren Reihen nebeneinander angeordnet sind. Eine so ausgebildete Mikrowellplatte kann dann beispielsweise in einem Brutschrank zur Zellkultivierung untergebracht sein, wobei das Anregungslicht und das Fluoreszenzlicht über Lichtwellenleiter von einer geeigneten Lichtquelle auf die Sensormembranen und das Fluoreszenzlicht von den Sensormembranen zu einem geeigneten optischen Sensor geführt werden kann. Dabei besteht die Möglichkeit, daß sowohl das Anregungslicht, wie auch das Fluoreszenzlicht durch einen einzigen Lichtwellenleiter geführt wird. Selbstverständlich kann auch eine entsprechende Lichtführung für die beiden verschiedenen Lichtarten in zwei gesonderten Lichtwellenleitern durchgeführt werden. Die Lichtwellenleiter müssen hierfür lediglich so fixiert und positioniert werden, daß ihre Aperturen eine optimale Fluoreszanzanregung und eine nahezu vollständige Erfassung des Fluoreszenzlichtes sichern. Dabei können zur Fixierung und Positionierung der Lichtwellenleiter gesonderte Halteplatten verwendet werden, die entsprechend der erfindungsgemäß zu verwendenden Gefäße dimensioniert und ausgerichtet werden, so daß die Lichtwellenleiter in bezug zu den Sensormembranen angeordnet und ausgerichtet sind. Diese Ausführung bietet sich insbesondere für mikrowellplattenförmig ausgebildete Gefäße an. Günstigerweise ist eine Kavität (Well) eines solchen mikrowellplattenförmigen Elementes für die bereits eingangs erwähnte Referenzmessung, d.h. lediglich mit flüssigem Medium, aber ohne Zellkulturen befüllt, verwendbar.

Die Ausführungsform für erfindungsgemäß ausgebildete und verwendbare mikrowellplattenförmige Elemente oder auch andere geeignete Gefäße können entsprechend der üblichen Laborstandards ausgebildet sein, so daß sie auch in herkömmlicher Form mit den verschiedenen bekannten Laborgeräten benutzt werden können.

Die ortsaufgelöste Messung verschiedener Proben kann aber nicht nur mit den den einzelnen Sensormembranen zugeordneten Lichtwellenleitern erfolgen, sondern es besteht außerdem die Möglichkeit eine Endoskopieanordnung zu verwenden, über die das mit ihr erfaßte Abbild einer größeren Anzahl von Sensormembranen auf beispielsweise eine CCD-Kamera gerichtet werden kann, so daß eine zeitgleiche ortsaufgelöste Messung der verschiedenen Sauerstoffkonzentrationen möglich wird.

Nachfolgend soll die Erfindung beispielhaft näher beschrieben werden.

Dabei zeigen:

Figur 1    eine schematische Darstellung eines Beispiels eines Gefäßes für die Überwachung der Stoffwechselaktivität von kultivierten Zellen in flüssigen Medien mit Sensormembranen und Lichtwellenleiter;

Figur 2    ein Diagramm mit dem schematisch die Sauerstoffkonzentration im flüssigen Medium vom Boden, auf dem sich die kultivierten Zellen befinden, des Gefäßes bis zur Oberfläche des Mediums, das im Gefäß enthalten ist, unter drei verschiedenen Bedingungen, wiedergegeben ist;

Figur 3    schematische Darstellungen von podestartigen Elementen mit sauerstoffsensitiven Membranen und alternativ einer zusätzlichen Oxidoreductase/Membran;

Figur 4    schematisch eine Möglichkeit für einen optischen Meßaufbau zur Fluoreszenzsignal-Erzeugung und Detektion an einer Vorrichtung gemäß Figur 1;

Figur 5    eine schematische Darstellung eines weiteren Beispiels eines Gefäßes mit sauerstoffpermeabler Abdeckung zur Überwachung der Stoffwechselaktität von kultivierten Zellen in flüssigen Medien;

Figur 6    ein Diagramm mit dem die Sauerstoffkonzentration im flüssigen Medium vom Boden, auf dem sich die kultivierten Zellen befinden, eines mit einer sauerstoffdurchlässigen Membran abgedeckten Gefäßes, unter drei verschiedenen Bedingungen, bis zur Oberfläche eines flüssigen Mediums, und weiter bis zur Umgebung in der das Gefäß enthalten ist, wiedergegeben ist;

Figur 7    eine schematische Darstellung eines in einem Brutschrank aufgenommenen Gefäßes, gemäß Figur 1;

Figur 8    ein Multigefäß in Verbindung mit einer Halteplatte für Lichtwellenleiter, die an eine Vorrichtung gemäß Figur 4 angeschlossen sind;

Figur 9      eine schematische Darstellung für eine Multiplexerfassung mehrerer Proben;

Figur 10      ein Beispiel einer erfindungsgemäßen Vorrichtung, bei der eine Multiwellplatte mittels einer abbildenden, z.B. Endoskopieanordnung, Optik überwacht wird;

Figur 11      den optischen Teil einer Vorrichtung gemäß Figur 10 zur Fluoreszenzsignal Erzeugung und Detektion;

Figur 12      ein Beispiel eines optischen Systems zur Fluoreszenzsignal Erzeugung und Detektion an einer Multiwellplatte zur gleichzeitigen Überwachung mehrerer Zellkulturen.

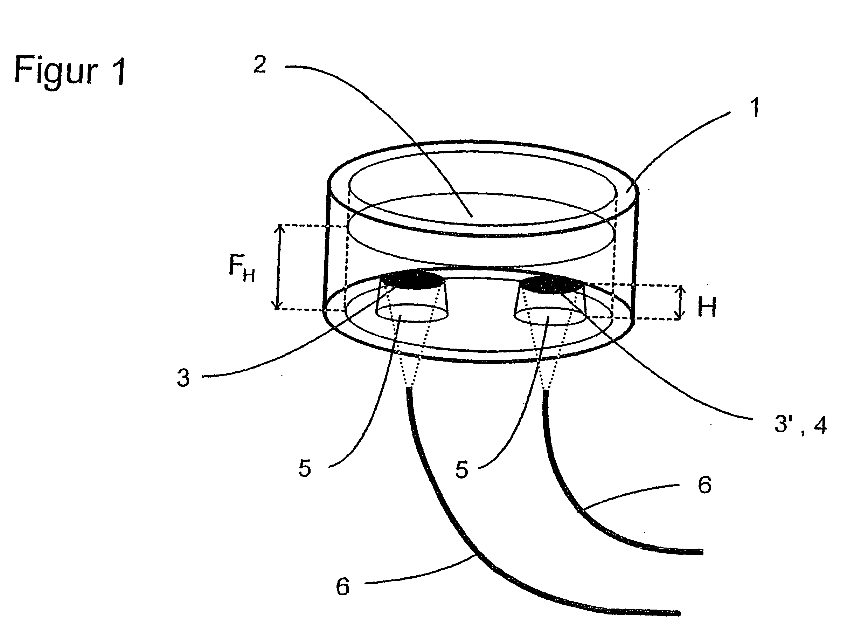

In der Figur 1 ist eine schematische Darstellung eines Beispiels eines Gefäßes 1 für die Überwachung der Stoffwechselaktivität von Zellkulturen in flüssigen Medien 2 gezeigt.

Das hier dargestellte Gefäß 1 ist überwiegend zylinderförmig ausgebildet hat eine optisch transparente Bodenplatte und ist an seiner Oberseite offen und demzufolge dort auch für Sauerstoff transparent ins flüssiges Medium 2 durchlässig. Das flüssige Medium 2 ist mit einer bestimmten Füllhöhe $F_H$ befüllt, in dem die Zellen kultiviert werden können. Am Boden des Gefäßes 1 sind bei diesem Beispiel zwei podestartige Elemente 5, aus einem optisch transparenten Material ausgebildet, deren obere Stirnflächen in einem bestimmten Abstand $\underline{H}$ von der Bodenfläche des Gefäßes 1 angeordnet sind. Dabei ist H generell kleiner als $F_H$

Auf der Stirnfläche des linken podestartigen Elements 5 ist eine Sensormembran 3 für die ausschließliche Messung der Sauerstoffkonzentration im flüssigen Medium 2 ausgebildet.

Auf der Stirnfläche des rechten podestartigen Elements 5 ist auf eine sauerstoffsensitive Sensormembran 3' zusätzlich eine Oxidoreductase Membran 4 ausgebildet.

Unterhalb des Gefäßes 1 sind jeweils für eine der Sensormembranen 3 und 3' Lichtwellenleiter 6, mit deren auf die Sensormembranen 3 ausgerichteten Lichtstrahlen (gestrichelt dargestellt) angeordnet. Durch die Lichtwellenleiter 6 kann Licht zur Fluoreszenzanregung eines in den Sensormembranen 3 und 3' enthaltenen fluoreszierenden Farbstoffes auf die Sensormembranen 3 und 3' gerichtet werden. Gegenläufig kann das Fluoreszenzlicht der Sensormembranen 3 und 3' durch die Lichtwellenleiter 6 aufgefangen werden.

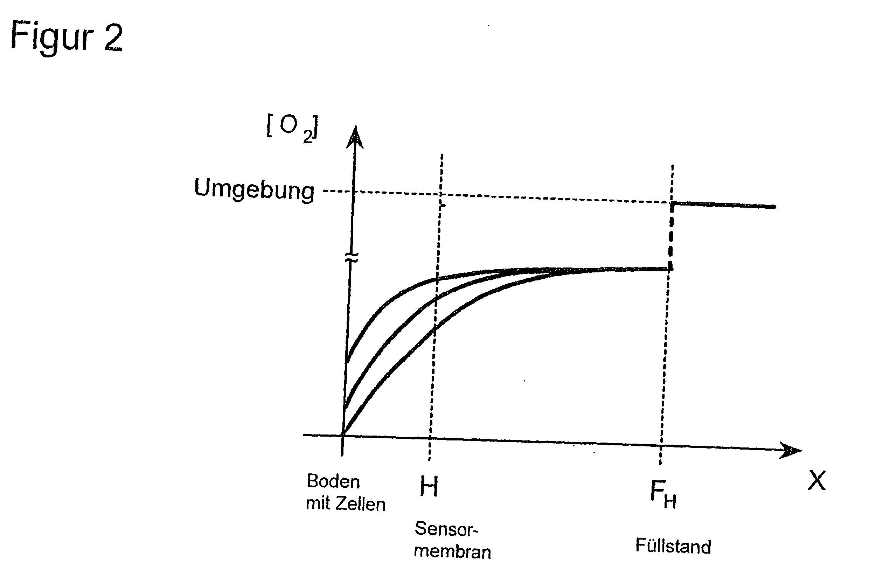

In der Figur 2 ist in einem Diagramm die Sauerstoffkonzentration im flüssigen Medium 2 mit am Boden befindenden kultivierten Zellen, ausgehend vom Boden eines Gefäßes 1 bis zur Oberfläche des flüssigen Mediums 2, unter drei verschiedenen Bedingungen, im Gefäß 1 dargestellt. Die unterste Kurve zeigt den Verlauf bei hohem Sauerstoffkonsum der kultivierten Zellen auf dem Boden und die oberste Kurve bei geringem Sauerstoff.

Dem Diagramm ist deutlich zu entnehmen, daß mit einer oberhalb des Gefäßbodens angeordneten Sensormembran 3 wesentlich höhere Werte einer Sauerstoffkonzentration im flüssigen Medium gemessen werden können, wohingegen die Sauerstoffkonzentration am Boden eines Gefäßes 1 gegen null strebt. Dies ist durch den Sauerstoffkonsum der kultivierten Zellen bedingt. Es wird so deutlich, daß eine Messung am Boden in unmittelbarer Nähe zu den Zellen nicht sinnvoll ist. Außerdem gibt dieses Diagramm den Sachverhalt wieder, daß bei einem Sauerstoffverbrauch der kultivierten Zellen mit einer Sensormembran oberhalb des Bodens eine Nachführung von Sauerstoff in das flüssige Medium 2 über deren Oberfläche bis zu den Zellen hin erfaßt werden kann. Es wird so deutlich, daß eine Sauerstoffmessung nur sinnvoll an einer Position zwischen den kultivierten Zellen und den Orten, an denen der Sauerstoff in das flüssige Medium 2 gelangt, ist.

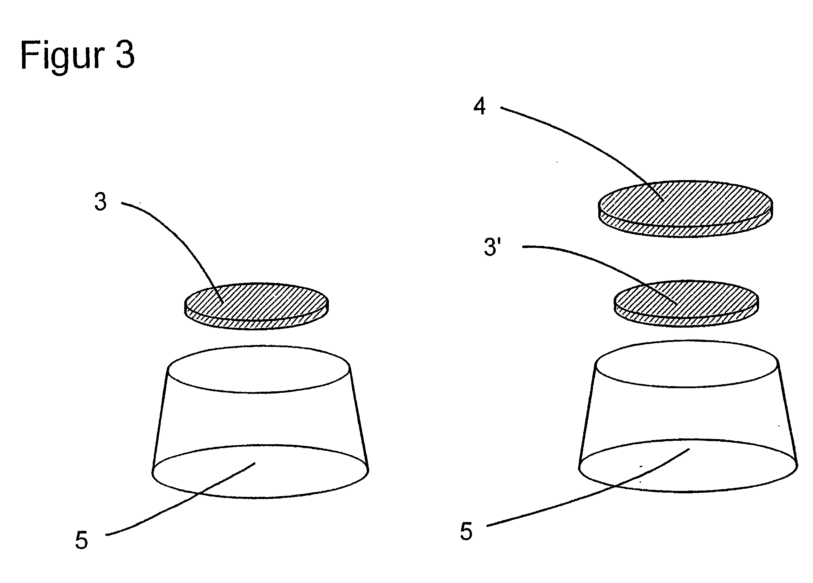

In der Figur 3 ist schematisch die Ausbildung podestartiger Elemente 5 dargestellt, wobei die linke Darstellung ein podestartiges Element 5 zeigt, das ausschließlich mit einer sauerstoffsensitiven Membran 3 versehen ist. Das podestartige Element 5 ist hier kegelstumpfförmig ausgebildet und besteht aus einem transparenten und für Sauerstoff zumindest nahezu undurchlässigem Material, mit dem erreicht werden kann, daß das Meßergebnis nicht durch über das Material eindringenden Sauerstoff verfälscht wird.

Analog hierzu ist auch das in Figur 3 rechts dargestellte podestartige Element 5 ausgebildet, wobei lediglich über der sauerstoffsensitiven Membran 3' eine zusätzliche Oxidoreductase-Membran 4 vorhanden ist.

Selbstverständlich sind die Membranen 3, 3' und 4 nicht, wie hier dargestellt, oberhalb, sondern unmittelbar auf den podestartigen Elementen 5 ausgebildet. Dabei überdeckt die Oxidoreductase-Membran 4 die sauerstoffsensitive Membran 3'.

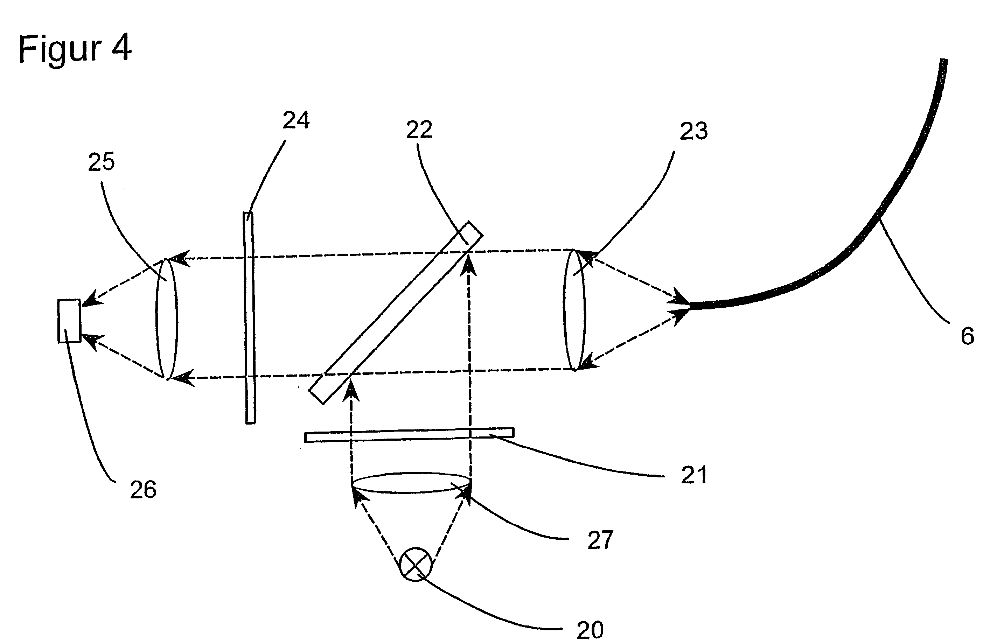

In der Figur 4 ist schematisch ein optischer Aufbau dargestellt, mit dem über einen Lichtwellenleiter 6 das Licht einer Lichtquelle 20 auf eine, hier nicht dargestellte, sauerstoffsensitive Membran 3 in einem Gefäß 1 bzw. einer Kavität 7, 7' gerichtet werden kann. Die Lichtquelle 20 strahlt bevorzugt nahezu monochromatisches Licht einer fluoreszenzanregenden Lichtwellenlänge über eine entsprechende Linse 27, gegebenenfalls einen Filter 21, der im wesentlichen Licht mit der Anregungslichtwellenlänge durchläßt auf einen dichroidischen Strahlteiler 22. Das Licht wird von dort über eine Linse 23 in den Lichtwellenleiter 6 einkoppelt. Selbstverständlich können auch andere bekannte Einkoppeloptiken für Lichtwellenleiter 6 Verwendung finden. Es kann auch eine multispektrale Lichtquelle in Verbindung mit einem geeigneten Filter verwendet werden, wobei ausschließlich der Filter die Monochromatisierung bewirkt.

Das Fluoreszenzlicht gelangt dann gegenläufig zum Anregungslicht durch den Lichtwellenleiter 6 über die Linse 23, einen Strahlteiler 22, durch einen entsprechend wellenlängenselektierten Filter 24, der nur Fluoreszenzlicht passieren läßt, gegebenenfalls über eine weitere Linse 25 auf einen optischen Detektor 26, mit dem die Intensität des Fluoreszenzlichtes, in Abhängigkeit von der jeweiligen Sauerstoffkonzentration gemessen werden kann.

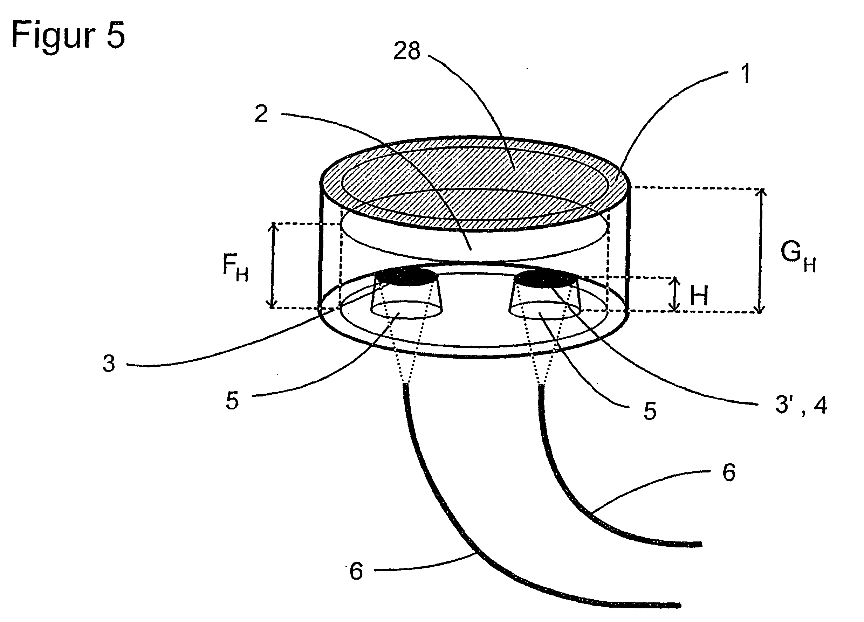

Das in der Figur 5 dargestellte Gefäß 1, entspricht in wesentlichen Punkten, dem bereits in Figur 1 dargestellten und entsprechend beschriebenen Gefäß.

Es ist lediglich mit einer Abdeckung 28 versehen, die zwar für Sauerstoff durchlässig ist, jedoch Kontaminationen verhindert und demzufolge die Sterilität sichert. Außerdem kann mit einer solchen Abdeckung 28 das Austrocknen des Gefäßes 1 verhindert werden.

In Figur 5 ist außerdem erkennbar, daß sich der Füllstand $F_H$ unterhalb der Gefäßhöhe $G_H$ befindet. $G_H$ gibt gleichzeitig den Abstand der Abdeckung 28 des Gefäßes 1 vom Boden wieder.

Durch die Abdeckung 28 wird der Sauerstoffgradient beeinflußt.

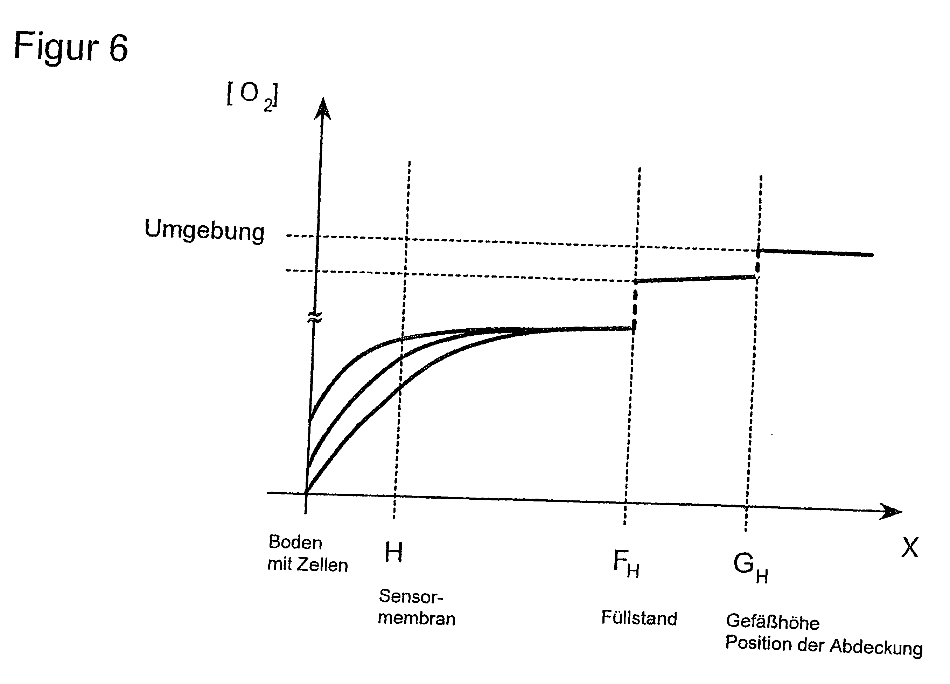

Dieser Sachverhalt ist in dem Diagramm, gemäß Figur 6 wiedergegeben und es wird deutlich gemacht, daß die Sauerstoffkonzentration zwischen Füllstandshöhe $F_H$ und Gefäßhöhe $G_H$ ebenfalls einen bestimmten Sauerstoffkonzentrationsgradienten, der durch die Abdeckung 28 hervorgerufen wird, bei Sauerstoffverbrauch durch Stoffwechselaktivität unterliegt.

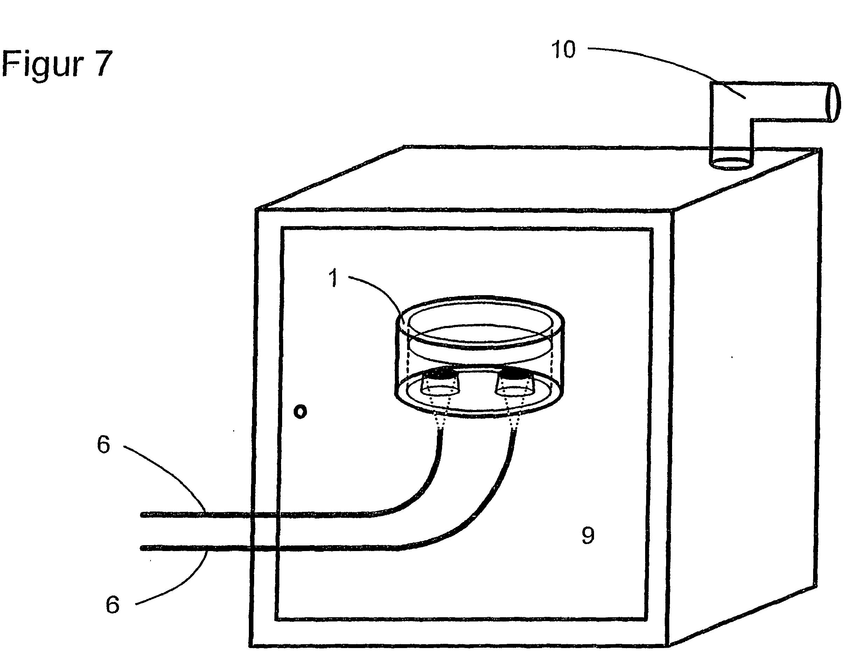

Bei dem in Figur 7 gezeigten Beispiel ist ein Gefäß 1 nach Figur 1 bzw. 5 in einem typischen Brutschrank 9 aufgenommen worden, in dem bekanntermaßen besonders optimale Bedingungen für die Zellkultivierung eingehalten werden können. Unter optimalen Bedingungen wird allgemein eine Temperatur von ca. 37 °C, eine relative Luftfeuchtigkeit von 100 % bei normalem atmosphären Druck und einem Sauerstoffpartialdruck im Gasraum, der der Umgebungsluft entspricht, verstanden. Die Lichtwellenleiter 6 für die beiden Sensormembranen 3 können aus dem Brutschrank 9 herausgeführt werden, so daß eine lokale Trennung von Meßort und Meßwerterfassung erreicht werden kann. In dieser Figur ist außerdem eine Gaszufuhr 10 für den Brutschrank 9 dargestellt, durch die beispielsweise atmosphärische Luft mit entsprechendem konstantem Sauerstoffgehalt nachgeführt werden kann. Brutschränke der beschriebenen Art werden standardgemäß zur Kultivierung von Zellen eingesetzt.

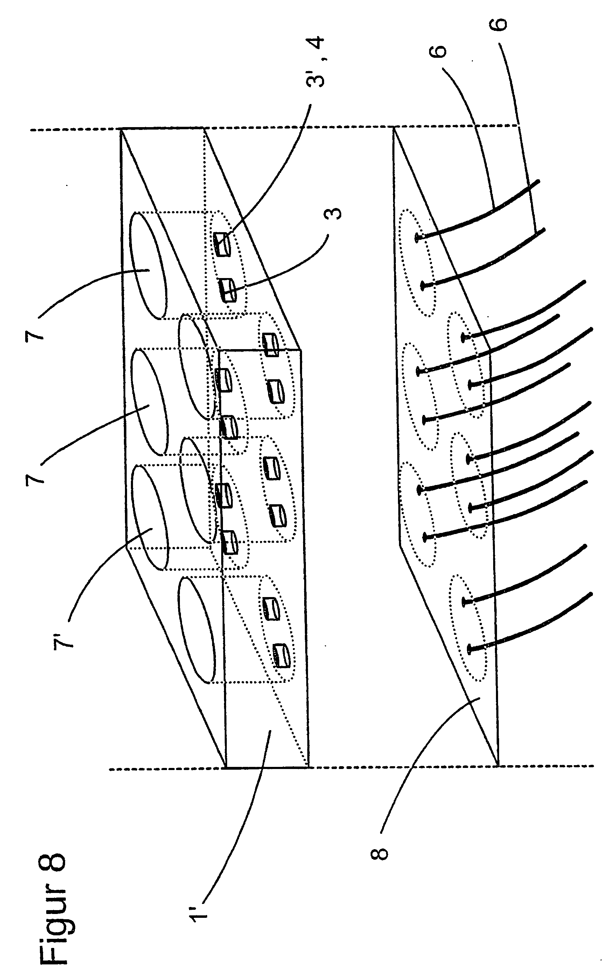

In der Figur 8 ist ein Beispiel für ein erfindungsgemäß zu verwendendes Gefäß 1' dargestellt, das als Multiwellplatte, wie diese sehr häufig zur Kultivierung von Zellen mit einer Mehrzahl von Kavitäten 7, 7' ausgebildet ist. In den Kavitäten 7, 7' sind jeweils wieder zwei Sensormembranen 3 und 3', wobei jeweils die Sensormembran 3' zusätzlich mit einer Oxidoreductase-Membran 4 versehen ist, analog zum Gefäß 1, wie es in Figur 1 beschrieben und gezeigt worden ist, angeordnet und ausgebildet.

Eine solche Multiwellplatte 1' kann wiederum in einem Brutschrank 9 aufgenommen werden und die Zellkulturen in den Kavitäten 7 entsprechend kultiviert werden. Im Brutschrank 9 kann unterhalb der Multiwellplatte 1' eine Halteplatte 8 angeordnet werden, mit der die Lichtwellenleiter 6 fixiert und positioniert werden können. Dabei sind die Lichtwellenleiter 6 an der Halteplatte 8 entsprechend der Anordnung der Sensormembranen 3 und 3' in den Kavitäten 7 und 7' des Gefäßes 1' gehalten. Die Halteplatte 8 kann dann im Brutschrank 9 in einem solchen Abstand zur Multiwellplatte 1' und hier insbesondere zu dessen Bodenfläche angeordnet werden, daß die Lichtstrahlen aus den Lichtwellenleitern 6 den gesamten Flächenbereich der jeweiligen, ihnen zugeordneten Sensormembranen 3 und 3' erfassen können. Die Verwendung der Halteplatte 8 erlaubt einen für die Zellkultivierung ungestörten Betrieb. Im Brutschrank werden die Zellen keiner weiteren Behandlung unterzogen. Zu solchen Zwecken wird die Multiwellplatte 1' aus dem Brutschrank und damit aus der Halterung 8 entnommen. Außerhalb des Brutschrankes kann dann die Behandlung der Zellkulturen im üblichen Sinne, wie z.B. Wechsel des flüssigen Mediums 2 oder Beobachtung der Zellen mit einem Mikroskop, ohne Einschränkung erfolgen.

Zumindest eine Kavität 7' einer solchen Multiwellplatte 1' kann für die bereits erklärte Referenzmessung verwendet werden, indem diese Kavität 7' ohne Zellen nur mit flüssigem Medium 2 befüllt wird.

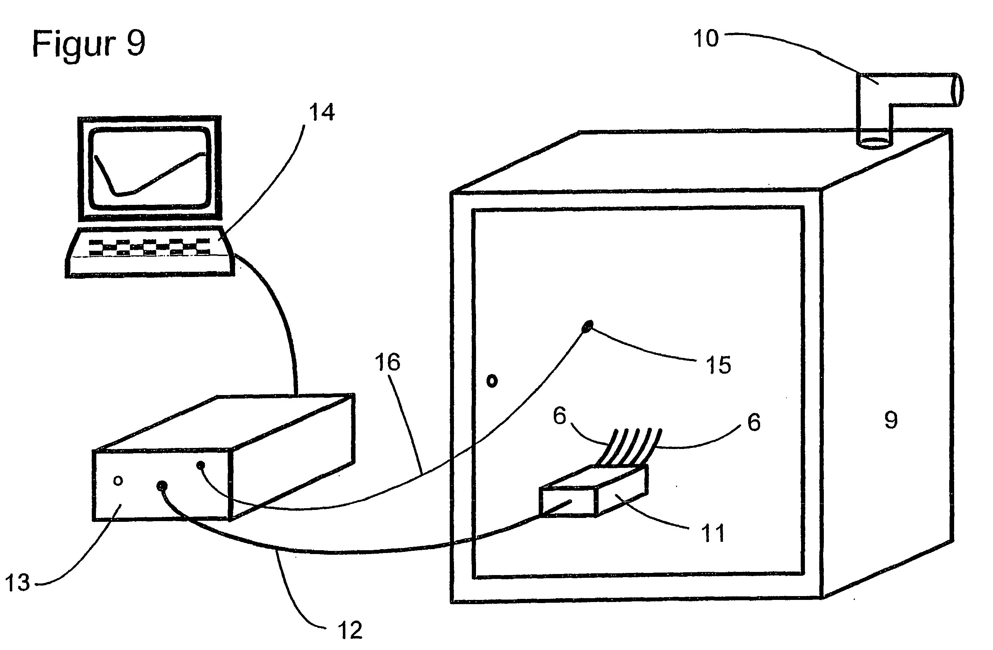

In der Darstellung nach Figur 9 ist schematisch ein optischer Multiplexbetrieb, mit dem neben der zeitauch eine ortsaufgelöste Messung mehrerer Proben, die in verschiedenen Gefäßen 1 bzw. Kavitäten 7 und 7' enthalten sind, möglich ist, gezeigt. Figur 9 zeigt damit einen komplexen Aufbau zur Durchführung des erfindungsgemäßen Verfahrens.

Eine Anordnung aus Lichtquelle 20, Detektor 26, Linsen 23, 25, 27, Filtern 21, 24 und Strahlteilern 22, wie in Figur 4 dargestellt, ist in einer Sende- und Meßeinheit 13 außerhalb eines Brutschrankes 9 angeordnet. Das Anregungslicht wird aus dieser Sende- und Meßeinheit 13 über einen einzigen Lichtwellenleiter 12 in einen optischen Multiplexer 11, an dem weitere Lichtwellenleiter 6 angeschlossen sind, geführt. Der optische Multiplexer 11 führt das Fluoreszenzanregungslicht sequentiell durch die einzelnen Lichtwellenleiter 6 zur Fluoreszenzanregung zu den verschiedenen Sensormembranen 3 und 3'.

Das Fluoreszenzlicht der verschiedenen Sensormembranen 3 und 3' gelangt wieder über die Lichtwellenleiter 6 zum optischen Multiplexer 11 und wird von diesem wiederum sequentiell, den jeweiligen Meßorten, also den jeweiligen Sensormembranen 3 und 3' entsprechend dem jeweiligen Probengefäß 7 bzw. 7' über den Lichtwellenleiter 12 auf einen optischen Detektor, der in der Einheit 13 enthalten ist, gerichtet. Die so ermittelten Signale können entsprechend orts- und zeitaufgelöst, zur Bestimmung der jeweiligen Sauerstoffkonzentration und der Oxidoreductase-Substratkonzentration genutzt werden.

Von der Einheit 13 gelangen die erfaßten Meßwerte zu einer Auswerte- und Steuereinheit 14, hier ein Personalcomputer, mit dem die Auswertung der erfaßten Meßsingnale und dabei insbesondere, der im allgemeinen Teil der Beschreibung bezeichnete Wertevergleich durch Messung am Referenzgefäß 7' durchgeführt werden kann. Mit der Auswerte- und Steuereinheit 14 können aber auch die Sende- und Meßeinheit 13 sowie der optische Multiplexer 11 gesteuert werden.

Außerdem sind im Inneren des Brutschrankes 9 Sensoren 15 für die Erfassung von Temperatur, relativer Feuchtigkeit, dem Gasdruck und gegebenenfalls der chemischen Zusammensetzung mit jeweiligem Partialdruck der beteiligten Gase der Gasatmosphäre angeordnet. Die von den Sensoren 15 erfaßten Meßwerte werden wiederum über eine gesonderte Leitung 16 in die Sende- und Meßeinheit 13 und von dort in die Auswerte- und Steuereinheit 14 geleitet, so daß damit der im allgemeinen Teil der Beschreibung bezeichnete Wertevergleich durchgeführt werden kann. Es besteht aber auch die Möglichkeit, die Leitung 16 unmittelbar von den Sensoren 15 zur Auswerte- und Steuereinheit 14 zu führen. Anhand der Sensordaten kann, wie im allgemeinen Teil der Beschreibung dargestellt, die Sauerstoffsättigungskonzentration im Referenzgefäß 7' berechnet werden und damit der Werteausgleich erfolgen.

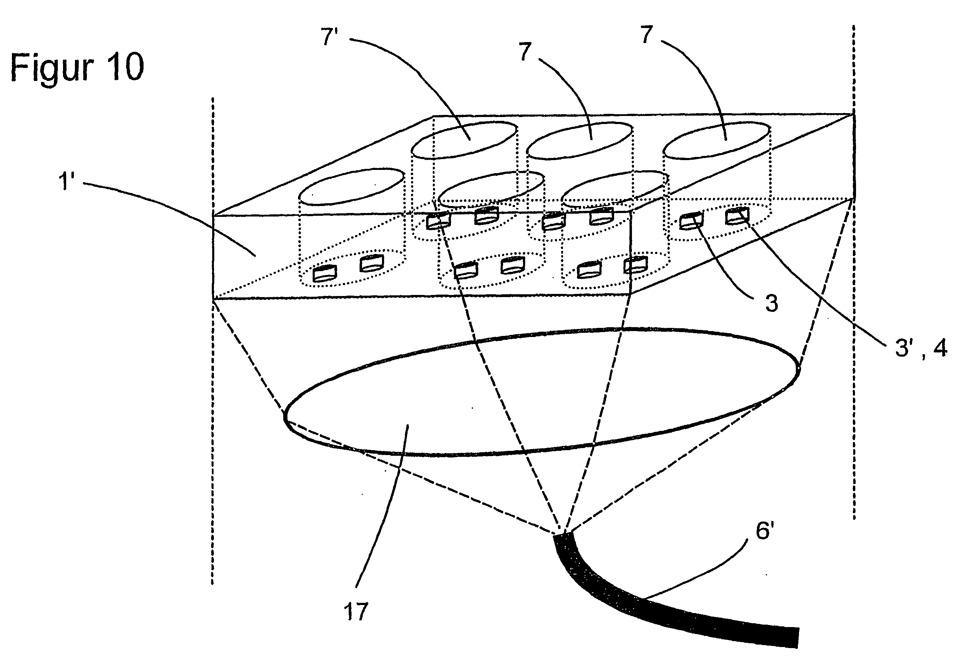

In der Figur 10 ist ein Beispiel zur gleichzeitigen. Überwachung mehrerer Proben, die in einer Mehrzahl von Kavitäten 7 und 7' einer Multiwellplatte 1' enthalten sind, wiedergegeben. Dabei ist unterhalb der Multiwellplatte 1' eine fokussierende Linse 17 angeordnet, mit der zumindest der Teil der Multiwellplatte 1' auf dem die Membranen 3 und 3' angeordnet sind, einmal mit Anregungslicht bestrahlt und zum anderen, das jeweilige Fluoreszenzsignal als Abbildungen in ein hier als Lichtwellenleiterbündel 6' verwendeten Lichtleitsystems einkoppelbar ist, abgebildet und weitergeleitet werden kann.

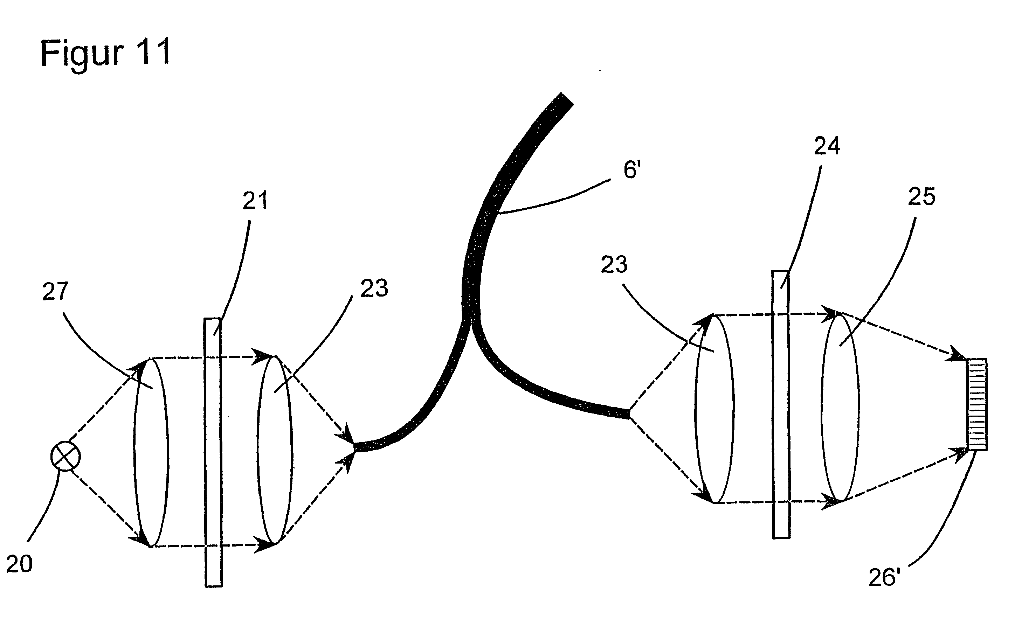

Die Fluoreszenzsignal Anregung und Detektion an den Membranen 3 und 3' in den Kavitäten 7 und 7' kann dann beispielhaft, wie in Figur 11 gezeigt, erfolgen. Hierzu wird wieder Licht einer Lichtquelle 20, über eine Linse 27, einen Filter 21 und eine Einkoppeloptik 23 in mindestens einem Teil des Lichtwellenleiterbündels 6' eingekoppelt und auf die Membranen 3 und 3', die wie bereits mehrfach beschrieben, auf podestartigen Elementen 5 in den Kavitäten 7 und 7' angeordnet sind, gerichtet, wobei durch den Filter 21 nur Licht aus der Lichtquelle 20 einer fluoreszenzanregenden Wellenlänge genutzt wird.

Das Fluoreszenzlicht der verschiedenen Membranen 3 und 3' gelangt wieder, wie hier schematisch dargestellt, aus dem anderen Teil des Lichtleiterbündels 6' über zwei Linsen 23, 25 und einen entsprechend geeigneten Filter 24 auf einen optischen Detektor 26', der hier eine für eine ortsaufgelöste Messung geeignete Kamera ist. Damit kann wieder ortsaufgelöst das Fluoreszenzsignal der einzelnen Membranen 3 und 3' der Kavitäten 7 und 7' erfaßt werden

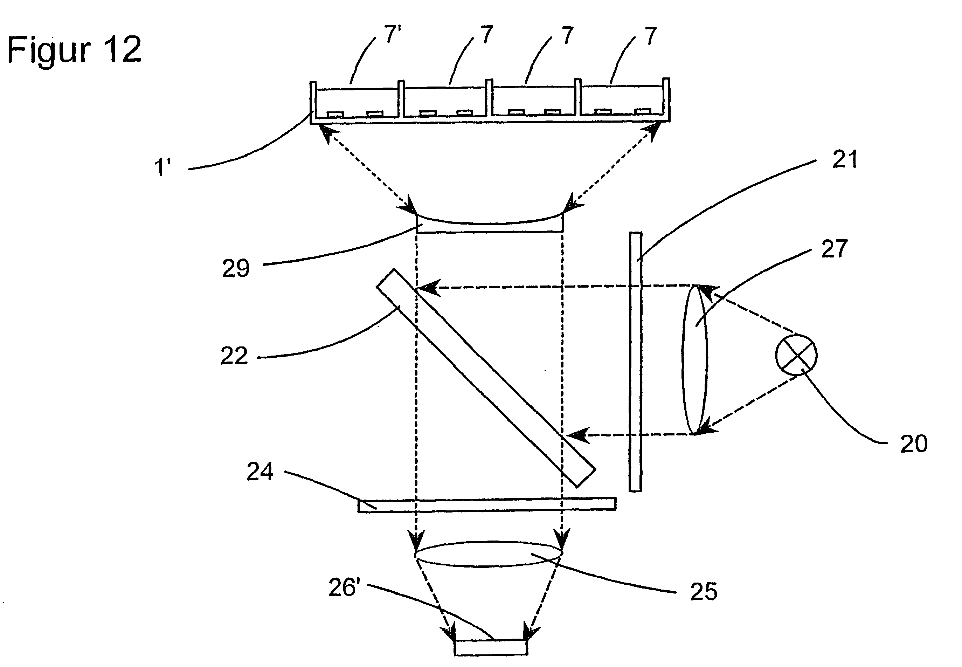

In der Figur 12 ist ein weiteres Beispiel eines optischen Aufbaus für eine gleichzeitige Überwachung verschiedener Proben, die in mehreren Kavitäten 7 und 7' einer Multiwellplatte 1' aufgenommen sind, dargestellt.

Dabei wird wieder Licht einer Lichtquelle 20 über einen Filter 21 zur Fluoreszenzanregung auf die verschiedenen sauerstoffsensitiven Membranen 3 und 3' durch den Boden des Gefäßes 1', der zumindest im Bereich der podestartigen Elemente 5 transparent ausgebildet ist, gerichtet. Zur Strahlformung und Strahlführung werden wieder verschiedene Linsen 27 und 29 sowie ein dichroidischer Strahlteiler 22 verwendet.

Das Fluoreszenzlicht der verschiedenen Membranen 3 und 3' gelangt über die Linse 29 durch den dichroidischen Strahlteiler 22 und gegebenenfalls einen geeigneten Filter 24 auf einen optischen Detektor 26', der hier wiederum eine für eine ortsaufgelöste Messung geeignete Kamera 26' sein kann. Die Abbildung des Fluoreszenzlichtes kann mittels einer zusätzlichen Linse 25 auf die Kamera 26' erfolgen.

**Patentansprüche**

1. Verfahren zur Überwachung der Stoffwechselaktivität von in flüssigen Medien kultivierten Zellen, wobei diese in für den Stofftransport von Sauerstoff in das flüssige Medium (2) teilweise durchlässigen Gefäßen (1, 7) aufgenommen sind, bei dem die Sauerstoffkonzentration optisch mit. Sensormembranen (3) im flüssigen Medium (2) zwischen den kultivierten Zellen und dem für den Sauerstofftransport in das flüssige Medium (2) maßgebend durchlässigen Teil des Gefäßes gemessen wird;
und die im flüssigen Medium (2) gemessene Sauerstoffkonzentration mit einem in einem nur mit flüssigem Medium ohne Zellen befüllten Vergleichsgefäß (7') gemessenen Konzentrationswert und/oder mittels einer mit Meßwerten anderer Parameter berechneten Sauerstoffkonzentration einem Wertevergleich unterzogen werden.

2. Verfahren nach Anspruch 1,
**dadurch gekennzeichnet,**
**daß** die Temperatur des flüssigen Mediums (2) im Gefäß (1, 7) und/oder einem Vergleichsgefäß (7') bestimmt wird und die relative Feuchtigkeit und der Druck außerhalb der Gefäße (1, 7, 7') gemessen und mit den jeweiligen Meßwerten eine dem entsprechende Sauerstoffkonzentration berechnet wird.

3. Verfahren nach Anspruch 2,

**dadurch gekennzeichnet, daß** die Temperatur des flüssigen Mediums (2) direkt in den Gefäßen (1, 7, 7') gemessen wird.

4. Verfahren nach Anspruch 2,
**dadurch gekennzeichnet, daß** indirekt über die Umgebungstemperatur die Temperatur in den Gefäßen (1,7, 7') gemessen wird.

5. Verfahren nach Anspruch 1 oder 2,
**dadurch gekennzeichnet, daß** bei variabler chemischer Zusammensetzung der Umgebungsatmosphäre der Gefäße (1, 7, 7'), deren Zusammensetzung mit den jeweiligen Stoffkonzentrationen bestimmt wird.

6. Verfahren nach einem der Ansprüche 1 bis 5,
**dadurch gekennzeichnet, daß** mit einem zweiten mit einer geeigneten Oxidoreductase (4) beschichteten Sauerstoff-Sensormembran (3') in einer Position zwischen den kultivierten Zellen und den für den Sauerstofftransport maßgebend durchlässigen Teil des Gefäßes im flüssigen Medium (2) die Konzentration des Substrates der Oxidoreductase im flüssigen Medium (2) bestimmt wird.

7. Verfahren nach Anspruch 6,
**dadurch gekennzeichnet, daß** die mit der ersten (3) und zweiten (3') Sauerstoffsensor-Membran in einem Gefäß (1, 7, 7') gemessenen Sauerstoffkonzentrationen, zur Bestimmung der Oxidoreductase-Substratkonzentration miteinander in Beziehung gesetzt werden.

8. Verfahren nach einem der Ansprüche 1 bis 7,
**dadurch gekennzeichnet, daß** die Stoffwechselaktivität der kultivierten Zellen über eine zeitaufgelöste Messung der Sauerstoffkonzentration mit dem daraus bestimmten Sauerstoffverbrauch der kultivierten Zellen und/oder der Oxidoreductase-Substratkonzentrationsänderung überwacht wird.

9. Verfahren nach einem der Ansprüche 1 bis 8,
**dadurch gekennzeichnet, daß** an den Sensormembranen (3, 3') die Fluoreszenzintensität oder Fluoreszenzabklingzeit eines in den Sensormembranen (3) enthaltenen fluoreszierenden Stoffes, bei dem eine der Sauerstoffkonzentration entsprechende Fluoreszenzlöschung zu verzeichnen ist, gemessen wird.

10. Verfahren nach einem der Ansprüche 1 bis 9,
**dadurch gekennzeichnet, daß** die Sauerstoffkonzentration in flüssigen Medien (2) mehrerer Gefäße (1, 7, 7') ortsaufgelöst gemessen wird.

11. Gefäß zur Überwachung der Stoffwechselaktivität von kultivierten Zellen in flüssigen Medien, das zumindest teilweise aus einem optisch transparenten Material besteht und teilweise für den Sauerstofftransport durchlässig ist und in dem mindestens eine Sensormembran zur optischen Messung der Sauerstoffkonzentration im flüssigen Medium angeordnet ist, **dadurch gekennzeichnet, daß** mindestens eine Sensormembran (3) oder (3') im Inneren des Gefäßes (1, 7, 7') zwischen den kultivierten Zellen und den für den Sauerstofftransport maßgebend durchlässigen Teil des Gefäßes im flüssigen Medium (2) angeordnet ist.

12. Gefäß nach Anspruch 11,
**dadurch gekennzeichnet,**
**daß** die Sensormembranen (3, 3' 4) an der Innenwandung und/oder auf am Gefäßboden angeordneten, zumindest teilweise transparenten, podestartigen Elementen (5) aufgebracht sind.

13. Gefäß nach Anspruch 11 oder 12,
**dadurch gekennzeichnet, daß** die Öffnung des/der Gefäße(s) (1, 7, 7') mit einer sauerstoffpermeablen Membran abgedeckt ist/sind.

14. Gefäß nach einem der Ansprüche 11 bis 13,
**dadurch gekennzeichnet, daß** mehrere Gefäße (7, 7') in Form einer Multiwellplatte (1') ausgebildet sind.

15. Vorrichtung zur Durchführung des Verfahrens nach einem der Ansprüche 1 bis 10, mit einem Gefäß nach einem der Ansprüche 11 bis 14,
**dadurch gekennzeichnet,**
**daß** über mindestens einen ersten Lichtwellenleiter (6) Licht einer nahezu monochromatischen Lichtquelle oder mit einem optischen Filter (21) entsprechend beeinflußtes Licht einer Lichtquelle (20) mit einer Fluoreszenz anregegenden Wellenlänge auf die Sensormembranen (3, 3') gerichtet ist.

16. Vorrichtung nach Anspruch 15,
**dadurch gekennzeichnet, daß** Fluoreszenzlicht über den ersten oder einen zusätzlichen zweiten Lichtwellenleiter (6) (über einen optischen Filter (24)) auf einen optischen Detektor (26) gerichtet ist.

17. Vorrichtung nach Anspruch 15 oder 16,
**dadurch gekennzeichnet, daß** den ersten und/oder zweiten, unterschiedlichen Sensormembranen zugeordnete Lichtwellenleiter (6) mit einem optischen Multiplexer (11) verbunden sind und der Multiplexer (11) mit einer nahezu monochromatischen Lichtquelle oder über einen Filter (21) mit einer Lichtquelle (20) und einem optischen Detektor (26) verbunden ist.

18. Vorrichtung nach einem der Ansprüche 15 bis 18,
**dadurch gekennzeichnet, daß** die Lichtwellenleiter (6) mittels einer Halteplatte (8) in bezug zu den Sensormembranen (3, 3') in den Gefäßen (1, 7, 7') positioniert sind.

19. Vorrichtung nach Anspruch 15, **dadurch gekennzeichnet, daß** über einen abbildenden Lichtwellenleiter (6'), ähnliche Lichtführungssystemen oder optischen Elementen (20, 21, 22, 23 27, 29) Licht einer nahezu monochromatischen Lichtquelle oder mit einem optischen Filter (21) entsprechend beeinflußtes Licht einer Lichtquelle (20) mit einer Fluoreszenz anregenden Wellenlänge auf die Sensormembran (3, 3') gerichtet ist.

20. Vorrichtung nach Anspruch 15, **dadurch gekennzeichnet, daß** die Sensormembran(en) (3, 3') mittels eines abbildenden Lichtleiters (6'), ähnlicher Lichtführungssysteme oder optische Elemente (22, 24, 25, 29) betrachtet und deren Abbildungen auf einer linearen oder flächenhaften Anordnung von lichtempfindlichen Detektoren (26') gerichtet sind.

21. Vorrichtung nach einem der Ansprüche 15 bis 20, **dadurch gekennzeichnet, daß** ein oder mehrere Gefäße (1, 7, 7') in einem Brutschrank (9) aufgenommen sind.

22. Vorrichtung nach einem der Ansprüche 15 bis 21, **dadurch gekennzeichnet, daß** in unmittelbarer Nähe zu den Gefäßen (1, 7, 7') oder im Brutschrank (9) Sensoren (15) zur Bestimmung der Temperatur, der relativen Feuchtigkeit und des Druckes der Gasatmosphäre vorhanden sind.

**Claims**

1. Method for monitoring the metabolic activity of cells cultivated in liquid media, in which the said cells are held in vessels (1, 7) which are partially permeable for the mass transfer of oxygen into the liquid medium (2), in the case of which method the oxygen concentration is measured optically with the aid of sensor membranes (3) in the liquid medium (2) between the cultivated cells and the part of the vessel which is decisively permeable for the oxygen transfer into the liquid medium (2); and the oxygen concentration measured in the liquid medium (2) is subjected to a comparison of values with a concentration value measured in a reference vessel (7') filled only with liquid medium without cells, and/or by means of an oxygen concentration calculated with the aid of measured values of other parameters.

2. Method according to Claim 1, **characterized in that** the temperature of the liquid medium (2) in the vessel (1, 7) and/or a reference vessel (7') is determined, and the relative humidity and the pressure outside the vessels (1, 7, 7') are measured and a corresponding oxygen concentration is calculated with the aid of the respective measured values.

3. Method according to Claim 2, **characterized in that** the temperature of the liquid medium (2) is measured directly in the vessels (1, 7, 7').

4. Method according to Claim 2, **characterized in that** the temperature in the vessels (1, 7, 7') is measured indirectly via the ambient temperature.

5. Method according to Claim 1 or 2, **characterized in that** in the event of a variable chemical composition of the ambient atmosphere of the vessels (1, 7, 7'), the composition of the latter is determined with the aid of the respective oxygen concentrations.

6. Method according to one of Claims 1 to 5, **characterized in that** a second oxygen sensor membrane (3'), coated with a suitable oxidoreductase (4), in a position between the cultivated cells and the part of the vessel decisively permeable for the oxygen transfer in the liquid medium (2) is used to determine the concentration of the substrate in the oxidoreductase in the liquid medium (2).

7. Method according to Claim 6, **characterized in that** the mutual relationship is found between the oxygen concentrations measured with the aid of the first (3) and second (3') oxygen sensor membranes in a vessel (1, 7, 7') in order to determine the oxidoreductase substrate concentration.

8. Method according to one of Claims 1 to 7, **characterized in that** the metabolic activity of the cultivated cells is monitored via a time-triggered measurement of the oxygen concentration with the aid of the oxygen consumption, determined therefrom, of the cultivated cells and/or change in the oxidoreductase substrate, concentration.

9. Method according to one of Claims 1 to 8, **characterized in that** the fluorescence intensity or fluorescence decay time of a fluorescent substance contained in the sensor membranes (3) in the case of which a fluorescence extinction corresponding to the oxygen concentration is to be noted is measured at the sensor membranes (3, 3').

10. Method according to one of Claims 1 to 9, **characterized in that** the oxygen concentration in liquid media (2) of a plurality of vessels (1, 7, 7') is measured in a spatially resolved fashion.

**11.** Vessel for monitoring the metabolic activity of cultivated cells in liquid media, which consists at least partially of an optically transparent material and is partially permeable for the oxygen transfer, and in which at least one sensor membrane for the optical measurement of the oxygen concentration is arranged in the liquid medium, **characterized in that** at least one sensor membrane (3) or (3') is arranged in the interior of the vessel (1, 7, 7') between the cultivated cells and the part of the vessel decisively permeable for the oxygen transfer in the liquid medium (2).

**12.** Vessel according to Claim 11, **characterized in that** the sensor membranes (3, 3', 4) are applied to the inner wall and/or pedestal-like elements (5) which are arranged on the vessel floor and are at least partially transparent.

**13.** Vessel according to Claim 11 or 12, **characterized in that** the opening of the vessel(s) (1, 7, 7') is covered by an oxygen-permeable membrane.

**14.** Vessel according to one of Claims 11 to 13, **characterized in that** a plurality of vessels (7, 7') are constructed in the form of a multiwell plate (1').

**15.** Device for carrying out the method according to one of Claims 1 to 10, having a vessel according to one of Claims 11 to 14, **characterized in that** light from a virtually monochromatic light source, or light, influenced appropriately by an optical filter (21), from a light source (20) of a wavelength exciting fluorescence is directed onto the sensor membranes (3, 3') via at least one first optical conductor (6).

**16.** Device according to Claim 15, **characterized in that** fluorescent light is directed onto an optical detector (26) via the first or an additional second optical conductor (6) (via an optical filter (24)).

**17.** Device according to Claim 15 or 16, **characterized in that** optical conductors (6) assigned to the first and/or second, different sensor membranes are connected to an optical multiplexer (11), and the multiplexer (11) is connected to a virtually monochromatic light source or, via a filter (21), to a light source (20) and an optical detector (26).

**18.** Device according to one of Claims 15 to 18, **characterized in that** the optical conductors (6) are positioned in the vessels (1, 7, 7') with reference to the sensor membranes (3, 3') by means of a holding plate (8).

**19.** Device according to Claim 15, **characterized in that** light from a virtually monochromatic light source, or light, influenced appropriately by an optical filter (21), from a light source (20) of a wavelength exciting fluorescence is directed onto the sensor membrane (3, 3') via a projecting optical conductor (6), similar light-guiding systems or optical elements (20, 21, 22, 23, 27, 29).

**20.** Device according to Claim 15, **characterized in that** the sensor membrane(s) (3, 3') is/are viewed by means of a projecting optical conductor (6), similar light-guiding systems or optical elements (22, 24, 25, 29), and their projections are directed onto a linear or planar arrangement of light-sensitive detectors (26').

**21.** Device according to one of Claims 15 to 20, **characterized in that** one or more vessels (1, 7, 7') are held in an incubator (9).

**22.** Device according to one of Claims 15 to 21, **characterized in that** sensors (15) for determining the temperature, the relative humidity and the pressure of the gas atmosphere are present in the immediate vicinity of the vessels (1. 7, 7') or in the incubator (9).

**Revendications**

**1.** Procédé de surveillance de l'activité métabolique de cultures cellulaires dans des milieux liquides, dans lequel celles-ci sont contenues dans des récipients (1, 7) partiellement perméables pour le transport de substances d'oxygène dans le milieu liquide (2), où la concentration en oxygène est mesurée par procédé optique avec des membranes de capteur (3) disposées dans le milieu liquide (2) entre les cultures cellulaires et la partie du récipient essentiellement perméable pour le transport d'oxygène dans le milieu liquide ;
et la concentration en oxygène mesurée dans le milieu liquide (2) est soumise à une comparaison de valeur avec une valeur de concentration mesurée dans un récipient comparatif (7') rempli uniquement avec le milieu liquide sans cellules et/ou au moyen d'une concentration d'oxygène calculée avec des valeurs mesurées d'autres paramètres.

**2.** Procédé selon la revendication 1, **caractérisé en ce que** l'on détermine la température du milieu liquide (2) dans le récipient (1, 7) et/ou un récipient comparatif (7'), et que l'on mesure l'humidité relative et la pression à l'extérieur des récipients (1, 7, 7'), puis que l'on calcule avec les valeurs mesurées respectives une concentration d'oxygène qui y correspond.

**3.** Procédé selon la revendication 2, **caractérisé en ce que** la température du milieu liquide (2) est mesurée directement dans les récipients (1, 7, 7').

**4.** Procédé selon la revendication 2, **caractérisé en ce que** l'on mesure la température dans les récipients (1, 7, 7') indirectement, par la température ambiante.

**5.** Procédé selon la revendication 1 ou 2, **caractérisé en ce que**, en cas de composition chimique variable de l'atmosphère environnante des récipients (1, 7, 7'), on détermine sa composition avec les concentrations de substances respectives.

**6.** Procédé selon l'une des revendications 1 à 5, **caractérisé en ce qu'**avec une seconde membrane de capteur d'oxygène (3') enrobée d'une oxydoréductase appropriée (4), dans une position entre les cultures cellulaires et la partie du récipient largement perméable pour le transport d'oxygène dans le milieu liquide (2), on détermine la concentration du substrat de l'oxydoréductase dans le milieu liquide (2).

**7.** Procédé selon la revendication 6, **caractérisé en ce que** les concentrations en oxygène mesurées dans un récipient (1, 7, 7') avec la première (3) et la seconde (3') membranes de capteur d'oxygène, sont mises en rapport les unes avec les autres pour déterminer la concentration du substrat en oxydoréductase.

**8.** Procédé selon l'une des revendications 1 à 7, **caractérisé en ce que** l'activité métabolique des cultures cellulaires est surveillée via une mesure en fonction du temps de la concentration en oxygène avec la consommation en oxygène déterminée à partir de celle-ci des cultures cellulaires et/ou de la modification de la concentration du substrat en oxydoréductase.

**9.** Procédé selon l'une des revendications 1 à 8, **caractérisé en ce qu'**au niveau des membranes de capteur (3, 3'), on mesure l'intensité de fluorescence ou l'extinction de la fluorescence d'une substance fluorescente contenue dans les membranes de capteur, où on enregistre une extinction de fluorescence qui correspond à la concentration en oxygène.

**10.** Procédé selon l'une des revendications 1 à 9, **caractérisé en ce que** l'on mesure localement la concentration en oxygène dans les milieux liquides (2) de plusieurs récipients (1, 7, 7').

**11.** Récipient de surveillance de l'activité métabolique de cultures cellulaires dans des milieux liquides, qui se compose au moins partiellement d'un matériau transparent sur le plan optique et qui est partiellement perméable pour le transport d'oxygène et dans lequel est disposé au moins une membrane de capteur permettant de mesurer par voie optique la concentration en oxygène dans le milieu liquide, **caractérisé en ce qu'**au moins une membrane de capteur (3) ou (3') est disposée à l'intérieur du récipient (1, 7, 7') dans le milieu liquide (2) entre les cultures cellulaires et la partie du récipient essentiellement perméable pour le transport d'oxygène.

**12.** Récipient selon la revendication 11, **caractérisé en ce que** les membranes de capteur (3, 3', 4) sont appliquées au niveau de la paroi interne et/ou sur des éléments (5) en forme de plate-forme disposés au niveau du fond du récipient et au moins partiellement transparents.

**13.** Récipient selon la revendication 11 ou 12, **caractérisé en ce que** l'ouverture du/des récipient(s) (1, 7, 7') est recouverte d'une membrane perméable à l'oxygène.

**14.** Récipient selon l'une des revendications 11 à 13, **caractérisé en ce que** plusieurs récipients (7, 7') sont formés sous la forme d'une plaque à plusieurs puits (1').

**15.** Dispositif de réalisation du procédé selon l'une des revendications 1 à 10 avec un récipient selon l'une des revendications 11 à 14, **caractérisé en ce que** l'on dirige sur les membranes de capteur (3, 3'), par le biais d'au moins une première fibre optique (6), de la lumière d'une source lumineuse presque monochromatique ou de la lumière d'une source lumineuse (20) influencée de façon correspondante avec un filtre optique (21), présentant une longueur d'onde excitant une fluorescence.

**16.** Dispositif selon la revendication 15, **caractérisé en ce que** la lumière de fluorescence est dirigée par la première ou par une seconde fibre optique supplémentaire (6) (à travers un filtre optique (24)) sur un détecteur optique (26).

**17.** Dispositif selon la revendication 15 ou 16, **caractérisé en ce que** les fibres optiques (6) coordonnées à la première et/ou à la seconde membrane(s) différente(s) sont reliées à un multiplexeur optique (11), et le multiplexeur (11) est relié à une source lumineuse presque monochromatique ou par le biais d'un filtre (21) à une source lumineuse (20) et à un détecteur optique (26).

**18.** Dispositif selon l'une des revendications 15 à 18, **caractérisé en ce que** les fibres optiques (6) sont positionnées au moyen d'une plaque de retenue (8) par rapport aux membranes de capteur (3, 3') des récipients (1, 7, 7').

**19.** Dispositif selon la revendication 15, **caractérisé en**

**ce que**, par une fibre optique d'imagerie (6'), des systèmes de guidage de lumière similaires où des éléments optiques (20, 21, 22, 23, 27, 29), on dirige sur la membrane de capteur (3, 3') de la lumière d'une source lumineuse presque monochromatique ou de la lumière influencée en conséquence avec un filtre optique (21) d'une source lumineuse (20) présentant une longueur d'onde excitant la fluorescence.

20. Dispositif selon la revendication 15, **caractérisé en ce que** la ou les membrane(s) de capteur (3, 3') sont examinées au moyen d'une fibre optique d'imagerie (6'), de systèmes de guidage de lumière similaires ou d'éléments optiques (22, 24, 25, 29) et que leurs images sont dirigées sur un dispositif linéaire ou plan de détecteurs photosensibles (26').

21. Dispositif selon l'une des revendications 15 à 20, **caractérisé en ce qu'**un ou plusieurs récipients (1, 7, 7') sont logés dans une chambre incubatrice (9).

22. Dispositif selon l'une des revendications 15 à 21, **caractérisé en ce que** sont présents, à proximité immédiate des récipients (1, 7, 7') ou dans la chambre incubatrice (9), des capteurs (15) permettant de déterminer la température, l'humidité relative et la pression de l'atmosphère gazeuse.

# Figur 1

EP 1 144 586 B1

Figur 2

# Figur 3

EP 1 144 586 B1

Figur 4

## Figur 5

EP 1 144 586 B1

Figur 6

[O$_2$]

Umgebung

Boden
mit Zellen

H

Sensormembran

F$_H$

Füllstand

G$_H$

Gefäßhöhe
Position der Abdeckung

X

EP 1 144 586 B1

Figur 7

Figur 8

7'   7   7

1'

3

3' , 4

8

6

6

EP 1 144 586 B1

Figur 9

Figur 10

Figur 11

EP 1 144 586 B1

Figur 12